# EUROPEAN PATENT APPLICATION

(11) **EP 4 647 485 A1**
(43) Date of publication of application: **12.11.2025**
(21) Application number: 25173782.1
(22) Date of filing: 30.04.2025
(51) Int. Cl.: C12M 1/107, C12M 1/00

(54) **CONCRETE TANK FOR FERMENTATION, METHOD OF MANUFACTURE AND USE OF SUCH CONCRETE TANK FOR FERMENTATION**

(30) Priority: 02.05.2024 BE 202405258
(71) Applicant: Bio-Dynamics nv, 8710 Wielsbeke (BE)
(72) Inventor: VAN DAMME, Thomas, 8710 Wielsbeke (BE)
(74) Representative: Brantsandpatents bv

(57) **Abstract**

The present invention relates to a concrete tank for fermentation comprising a concrete floor slab, a concrete side wall, and a concrete roof structure, wherein the concrete tank comprises a roof insulation layer, wherein the roof insulation layer is placed on an interior side of the concrete tank against the concrete roof structure. The invention also relates to a method for manufacturing a concrete tank for fermentation comprising the steps of casting a concrete floor slab, casting a concrete side wall, and casting a concrete roof structure, wherein after casting the concrete side wall, a formwork for casting the concrete roof structure is installed, wherein a roof insulation layer is installed in the formwork, after which the concrete roof structure is cast onto the roof insulation layer. Finally, the invention relates to the use of such a concrete tank for the fermentation of biomass.

## Description

### TECHNICAL FIELD

The invention relates to a concrete tank, more specifically a concrete tank for fermentation. In a second aspect, the invention also relates to a method for manufacturing such a concrete tank. In another aspect, the invention also relates to a use of the concrete tank for biomass fermentation.

### PRIOR ART

Concrete tanks as fermentation tanks are known from the prior art. Such a tank has a concrete floor slab, a concrete side wall and a concrete roof structure. Biomass is added to the concrete tank. An example of a suitable biomass is manure. The biomass ferments in the tank, forming digestate and mainly methane gas. This methane gas is usually used in a cogeneration plant for generating heat and electricity or purified and pumped into the gas network.

For a smooth fermentation process it is advisable to insulate the concrete tank. This not only concerns the concrete side wall, but also the concrete roof structure. The concrete roof structure is preferably constructed as lightly as possible. To insulate the concrete roof structure, insulated sandwich panels are installed on the concrete roof structure. Such sandwich panels are very light and suitable for installation on an exterior side of the concrete tank. A disadvantage of sandwich panels is that they cannot be walked on. Many fermentation tanks are equipped with an agitator that, for example, must be accessible from above for maintenance and repairs. It is therefore necessary to place a walkway above the sandwich panels, which increases the weight of the roof of the concrete tank. In addition, it is also necessary to protect an interior side of the concrete tank against sulfur or other harmful substances in the formed methane gas.

Document CN210264105U discloses a cast-in-place reinforced concrete tank, consisting of a foundation and a tank wall with a ring-shaped reinforcement frame placed on it. Concrete is applied around this reinforcement frame, whereby the use of corrugated inner and outer templates forms a corrugated inner and outer wall, respectively, to improve the structural strength of the tank. However, the document does not address the problem of gases that are toxic or can cause damage to components. In addition, the ribs in both the horizontal and vertical directions cause problems that can lead to increased material usage and structural weakening. Furthermore, the ridges in the wall result in uneven circulation and thus reduced homogeneity of the fermentation mixture, leading to less efficient fermentation. The curved roof structure causes practical and structural problems such as no stacking function or the possibility to walk on it.

Document CN101104840A discloses a biogas fermentation tank with low temperature tolerance, in which the tank wall consists of a cylindrical sandwich structure with a heat-insulating layer between an inner and outer wall. The small thickness of 3-5 cm will result in reduced performance. However, in this document, the insulation layer is not in contact with the interior of the fermentation tank, and the problem of gases that are toxic or can cause damage to components is not addressed.

Document CN201416006Y discloses a biogas installation constructed from steel mesh-cement panels that together form a box-shaped structure. In this document, the insulation layer is located on the outside of the tank and the problem of gases that are toxic or can cause damage to components is not addressed. In addition, the document shows a curved roof structure with many disadvantages.

The present invention aims to provide at least a solution for some of the above-mentioned problems or disadvantages.

### SUMMARY OF THE INVENTION

In a first aspect, the present invention relates to a concrete tank according to claim 1.

Placing the roof insulation layer on an interior side of the concrete tank is advantageous because it makes the roof of the concrete tank accessible without damaging the roof insulation. It is not necessary to install walkways on the roof of the concrete tank. The concrete roof structure can be walked on directly and the concrete tank can be constructed more simply. An additional advantage is that, depending on the chosen insulation material, the roof insulation layer already provides initial protection against sulfur or other similar harmful substances in the methane gas formed in the concrete tank. This embodiment is particularly advantageous because the plastic lining, regardless of the chosen insulation material, protects the concrete roof structure against sulfur or other similar harmful substances in the methane gas formed in the concrete tank. As a result, the concrete tank is particularly suitable for fermentation.

Preferred embodiments of the concrete tank are set out in claims 2 to 8.

In a second aspect, the present invention relates to a method according to claim 9.

This method has, among other things, the advantage that by placing the roof insulation layer in the formwork, no additional insulation, for example in the form of sandwich panels, needs to be installed on the concrete roof structure afterwards. As a result, it is also not necessary to install walkways on the roof of the concrete tank to make the roof accessible, further simplifying the construction of the concrete tank. An additional advantage is that, depending on the chosen insulation material, the roof insulation layer already provides initial protection against sulfur in the methane gas formed in the concrete tank.

Preferred embodiments of the method are described in dependent claims 10 to 14.

In a third aspect, the present invention relates to a use according to claim 15.

This use results in a cost-effective biomass fermentation in a concrete tank, whereby a high and stable temperature is obtained in the concrete tank due to the layer of roof insulation. As a result, little or no heat needs to be supplied to the concrete tank to maintain the fermentation or pre-acidification. A stable climate can be achieved in the concrete tank.

### DESCRIPTION OF THE FIGURES

**Figure 1** shows a cross-section in a vertical plane of a concrete roof structure of a concrete tank according to an embodiment of the present invention.
**Figure 2** shows a cross-section in a vertical plane of a concrete roof structure of a concrete tank according to an alternative embodiment of the present invention.
**Figure 3** shows a cross-section in a vertical plane of a concrete roof structure of a concrete tank according to an alternative embodiment of the present invention with plastic lining on the side wall with bonding mortar.
**Figure 4** shows a cross-section in a vertical plane of a concrete roof structure of a concrete tank according to an alternative embodiment of the present invention with plastic lining on the side wall with bonding mortar.
**Figure 5** shows a cross-section in a vertical plane of a concrete roof structure of a concrete tank according to an alternative embodiment with nails in the insulation and with plastic lining on the side wall.
**Figure 6** shows a cross-section in a vertical plane of a concrete roof structure of a concrete tank according to an alternative embodiment with nails in the insulation and with plastic lining on the side wall.
**Figure 7** shows a cross-section in a vertical plane of a concrete roof structure of a concrete tank according to an alternative embodiment with plastic lining on the side wall.

### DETAILED DESCRIPTION

Unless otherwise defined, all terms used in the description of the invention, including technical and scientific terms, have the meanings as commonly understood by a person skilled in the art to which the invention pertains. For a better understanding of the description of the invention, the following terms are explicitly explained.

In this document, "a" and "the" refer to both the singular and the plural, unless the context presupposes otherwise. For example, "a segment" means one or more segments.

The terms "comprise," "comprising," "consist of," "consisting of," "provided with," "include," "including," "contain," "containing," are synonyms and are inclusive or open terms that indicate the presence of what follows, and which do not exclude or prevent the presence of other components, characteristics, elements, members, steps, as known from or disclosed in the prior art.

Quoting numerical ranges by endpoints includes all integers, fractions and/or real numbers between the endpoints, these endpoints included.

The term plastic lining refers to a protective layer that is applied to protect the concrete roof structure and the underlying insulation layer against aggressive substances, such as sulfur-containing components in the methane gas formed in the tank. The plastic lining can take various forms and attachment methods, without being limited thereto. For example, the plastic lining can be implemented as a cast-in plastic lining, in which it is applied in the formwork before casting the concrete and, after curing, becomes an integral part of the concrete structure. In addition, the lining can also be applied in the form of a foil that is attached to the surface after casting the concrete, either by gluing or by mechanical fasteners such as nails. Furthermore, there are embodiments in which the protective layer is mechanically anchored, for example by using foils with protrusions or anchors that are fixed in an intermediate layer or in the insulation layer. An alternative approach consists of applying a coating, such as a liquid or paste-like substance that is spread over the concrete surface and, after curing, forms a protective film.

The term "protrusions" refers to structures located on one side of the plastic foil, opposite the smooth side. The smooth side forms the interior of the tank, while the protrusions are located on the side of the insulation layer or the concrete walls. "Protrusions" include, among others, hooks, loops, V-shaped structures, as well as a non-woven finish that creates a coarser structure. The protrusions serve to achieve improved bonding between the plastic foil and the insulation layer, or between the plastic foil and a concrete structure, such as a concrete side wall.

The term "sulfur" refers to the presence of sulfur in the form of methane gas components released during the fermentation process. However, sulfur is only one example of aggressive substances that may be present in the fermentation mixture. These substances can take various forms-gaseous, liquid, or solid-and include, among others, corrosive compounds, acids, and abrasive particles. The presence of such aggressive substances poses a risk to the integrity of the concrete fermentation tank, as they can attack the concrete and thus negatively affect the lifespan and safety of the installation.

In a first aspect, the invention relates to a concrete tank for fermentation.

The concrete tank comprises a concrete floor slab, a concrete side wall, and a concrete roof structure.

The concrete floor slab is preferably a disk-shaped floor slab. The concrete floor slab is preferably reinforced using reinforcement meshes. The reinforcement meshes have a mesh size of at most 150 mm, preferably at most 100 mm. The bars of the reinforcement meshes preferably have a diameter of at least 10 mm, more preferably at least 12 mm. Preferably, the floor slab comprises an upper reinforcement and a lower reinforcement. The upper reinforcement is preferably at a distance of at least 30 mm from a top side of the concrete floor slab, more preferably at least 40 mm. The upper reinforcement is preferably at a distance of at most 70 mm from the top side of the concrete floor slab, more preferably at most 60 mm, even more preferably at most 55 mm, and even more preferably at most 50 mm. Bars of reinforcement meshes of the upper reinforcement preferably have a diameter of at least 14 mm, more preferably at least 16 mm. The lower reinforcement is preferably at a distance of at least 30 mm from an underside of the concrete floor slab, more preferably at least 40 mm. The lower reinforcement is preferably at a distance of at most 70 mm from the underside of the concrete floor slab, more preferably at most 60 mm, even more preferably at most 55 mm, and even more preferably at most 50 mm. The concrete floor slab is placed directly on a ground surface or on a foundation. The foundation is a trench or pile foundation. Preferably, the foundation is a pile foundation.

The concrete side wall is preferably cast in place. The concrete side wall is preferably cylindrical. The concrete side wall is preferably cast layer by layer. The concrete side wall is preferably reinforced. The concrete side wall is reinforced using reinforcement meshes. Alternatively, the concrete side wall is reinforced with pre-tensioned or post-tensioned cables, wherein the pre-tensioned or post-tensioned cables are placed in circular loops in the concrete side wall. The cylindrical loops are concentric with a circumference of the concrete side wall. Alternatively, the concrete side wall is reinforced with a combination of at least two elements from a group consisting of reinforcement meshes, pre-tensioned cables, and post-tensioned cables. Preferably, the concrete side wall is reinforced at least with post-tensioned cables. The concrete side wall is preferably insulated. This is advantageous for stable conditions in the concrete tank for fermentation. The concrete side wall is optionally heated using piping in the cylindrical side wall.

The concrete roof structure is preferably reinforced. The concrete roof structure is preferably reinforced using reinforcement meshes. The reinforcement meshes preferably have a mesh size of at most 150 mm. The bars of the reinforcement meshes preferably have a diameter of at least 6 mm, more preferably at least 8 mm. Preferably, the concrete roof structure comprises an upper reinforcement and a lower reinforcement. The upper reinforcement is preferably at a distance of at least 20 mm from a top side of the concrete roof structure, more preferably at least 30 mm, and even more preferably at least 35 mm. The upper reinforcement is preferably at a distance of at most 70 mm from the top side of the concrete roof structure, more preferably at most 60 mm, even more preferably at most 50 mm, and even more preferably at most 45 mm. The lower reinforcement is preferably at a distance of at least 20 mm from an underside of the concrete roof structure, more preferably at least 30 mm, and even more preferably at least 35 mm. The lower reinforcement is preferably at a distance of at most 70 mm from the underside of the concrete roof structure, more preferably at most 60 mm, even more preferably at most 50 mm, and even more preferably at most 45 mm.

The concrete roof structure is a cast-in-place roof structure. For this, a temporary scaffolding is built inside the concrete tank to support a formwork for casting the concrete roof structure.

Optionally, the concrete tank includes an agitator suspended from the concrete roof structure.

According to a preferred embodiment, the concrete tank comprises a roof insulation layer. The roof insulation layer is placed on an interior side of the concrete tank against the concrete roof structure. As a result, the roof of the concrete tank is accessible without the roof insulation being damaged. It is not necessary to install walkways on the roof of the concrete tank. The concrete roof structure can be walked on directly and the concrete tank can be constructed more simply.

The roof insulation layer is, for example, constructed with pre-formed boards made of extruded polystyrene, expanded polystyrene, polyurethane, polyisocyanurate, or another suitable material. The panels are nailed, screwed, glued, or otherwise attached to the concrete roof structure. In an alternative preferred embodiment, the roof insulation layer is formed in place, as described in other embodiments. In this alternative preferred embodiment, the insulation can be formed using the concrete side wall as a mold for the insulation. By using the already present concrete side wall as formwork for spraying the roof insulation in place, the construction process is simplified, material and labor consumption is reduced, improved connection between insulation and structure is achieved, cutting losses are avoided, and solid support is provided against internal spray pressures. Moreover, the roof insulation can be mechanically anchored to the roof structure via lattice girders, while the plastic lining is anchored in the insulation layer without additional gluing or fastening thanks to the protrusions. More preferably, pre-formed boards are used, which have the advantage that installation is less weather-dependent than with cast-in-place variants. An additional advantage is that these do not require heavy equipment such as large pumps to install them at great height. Spraying and casting insulation material requires specialized equipment such as pumps, spray installations, and concrete mixers, as well as specific know-how, which is not always available. By using insulation boards, this complexity can easily be avoided. In addition, the transport of pre-formed boards is simple and these materials are available near the installations. Moreover, with spray or casting systems, a prior study is required to determine the placement of mechanical anchors, which leads to additional costs. Furthermore, mortars with EPS and other sprayed materials often have a lower insulation rating, especially when not applied correctly. In the present method, insulation boards manufactured in a controlled environment are used, allowing boards with predetermined and controlled specifications to be used, even in regions where sprayed insulation is not available. The installation of the insulation boards can also proceed more quickly and does not require drying times, as is the case with sprayed insulation materials. The boards can be installed with standard equipment already available on site for the construction of the fermentation tanks. As a result, there is no need for additional specialized equipment or infrastructure, resulting in efficient installation without extra material or financial investments. This feature makes the system particularly suitable for integration into existing construction processes, without significant impact on site planning or budget. Even more preferably, XPS with a waffled surface texture is used. XPS is moisture-resistant and the waffled structure ensures optimal adhesion with, on the one hand, the (adhesive) mortar between the insulation and the foil and, on the other hand, the concrete that is subsequently cast on top.

An additional advantage of this embodiment is that, depending on the chosen insulation material, the roof insulation layer already provides initial protection against sulfur or other aggressive substances in the methane gas formed in the concrete tank.

According to a preferred embodiment, a plastic lining is installed on the interior side of the concrete tank under the roof insulation layer to protect the concrete roof structure and the roof insulation layer. The plastic lining protects the concrete roof structure and the roof insulation layer at least against sulfur or other aggressive substances in the methane gas formed in the concrete tank. The plastic lining is, for example, in the form of a foil. The foil can be, without being limited thereto, one of the following types: a foil provided with large V-shaped protrusions that promote anchoring in the cast insulation layer; a foil provided with small loop-shaped protrusions that enable bonding with a (adhesive) mortar layer between the foil and the insulation boards or between the foil and a concrete intermediate layer; or a foil whose upper side is finished with a non-woven structure that also ensures bonding with the (adhesive) mortar or the concrete intermediate layer. The plastic lining is preferably at least 1.0 mm thick, more preferably at least 1.5 mm, and even more preferably at least 2.0 mm. The plastic lining is preferably at most 15 mm thick, more preferably at most 12 mm, even more preferably at most 11 mm, and even more preferably at most 10 mm. A non-limiting list of suitable materials for the plastic lining includes polyethylene, polyvinyl chloride, polypropylene. The plastic lining is glued to the roof insulation layer, screwed or nailed through the roof insulation layer to the concrete roof structure, or installed under the roof insulation layer by another suitable means. Alternatively, an additional concrete layer is provided between the roof insulation layer and the plastic lining, with the plastic lining preferably applied directly to the additional concrete layer. This is advantageous if the plastic lining is difficult to apply directly to the roof insulation layer.

This embodiment is particularly advantageous because the plastic lining, regardless of the chosen insulation material, protects the concrete roof structure against sulfur in the methane gas formed in the concrete tank. As a result, the concrete tank is particularly suitable for fermentation. During the fermentation process, various aggressive substances are released that may be present in solid, liquid, or gaseous form in the fermentation mixture and may be harmful to concrete components of the installation. Examples of solid aggressive substances include abrasive particles such as sand, indigestible fiber residues (for example, lignin), and small metal or glass fragments. Gaseous aggressive components may include hydrogen sulfide (H₂S), ammonia (NH₃), sulfur dioxide (SO₂), and other sulfur-containing compounds formed during the breakdown of organic sulfur compounds. In liquid form, aggressive substances may include organic acids such as acetic acid or butyric acid, as well as fatty acids or dissolved salts such as ammonium, sulfate, or other sulfur compounds in aqueous solution. These substances can cause chemical degradation, corrosion, or erosion of the concrete, which adversely affects the structural integrity of the tank.

According to a further embodiment, the plastic lining comprises protrusions. Non-limiting examples of suitable forms for the protrusions are hooks, loops, V-shapes, or a non-woven finish. In a further embodiment, the plastic lining preferably comprises a flat side and a side with a non-woven finish. The protrusions engage in the roof insulation layer. The side with protrusions is particularly advantageous for good bonding of the plastic lining in the roof insulation layer, especially when the roof insulation layer is formed in place or in the additional concrete layer. In an alternative embodiment, the plastic lining is first tensioned in the formwork before casting the concrete. For example, when the roof insulation layer is formed in situ, the plastic lining is placed in formwork for the concrete roof structure, after which, before casting the concrete roof structure, the roof insulation layer or the additional concrete layer is first formed. The protrusions automatically engage in the roof insulation layer or the additional concrete layer when forming the roof insulation layer. As a result, no additional steps are required, such as nailing, screwing, gluing, etc., to install the plastic lining under the roof insulation layer. Pre-installing the plastic lining before casting the concrete ensures robust and durable anchoring in the concrete wall. Moreover, this method makes the installation independent of weather conditions, as it takes place inside the formwork, and reduces labor intensity compared to gluing or mechanically fastening the lining after the concrete has cured. A rubber layer or a coating could also be suitable for protecting the roof insulation layer and the concrete roof structure against sulfur or other substances in the formed methane gas. This alternative has the disadvantage that the rubber layer or coating can only be applied after casting the concrete roof structure. The formwork for casting must first be removed, and only then can the rubber layer or coating be applied. Applying the rubber layer or coating is also more labor-intensive. Another disadvantage of applying a rubber layer or coating is that favorable weather conditions are necessary. An additional advantage of a plastic lining is that it has a very long service life of more than one hundred years, whereas a rubber layer or a coating must be repaired or renewed on average every ten years.

According to a preferred embodiment, the roof insulation layer comprises spacers. The spacers are preferably lattice girders. Spacers are used in concrete reinforcement to maintain the correct distance between reinforcement layers. For example, when casting a concrete floor, spacers are placed between a lower reinforcement and an upper reinforcement. Non-limiting examples of suitable lattice girders are support girders, A-girders, rebar chairs, zigzag girders, etc. The spacers in the roof insulation layer protrude above the roof insulation layer into the concrete roof structure. The spacers preferably protrude at least 20 mm above the roof insulation layer, more preferably at least 25 mm, even more preferably at least 30 mm, even more preferably at least 35 mm, and most preferably at least 40 mm.

This embodiment is particularly advantageous in combination with a roof insulation layer formed in place. Because the spacers in the roof insulation layer protrude into the concrete roof structure, after casting the concrete roof structure, the roof insulation layer will automatically be placed against the concrete roof structure. As a result, no additional steps are required, such as gluing, nailing, or screwing, to place the roof insulation layer against the concrete roof structure, which simplifies the construction of the concrete tank. The primary function of the spacers in the roof insulation layer is to suspend the roof insulation layer from the concrete roof structure. It is therefore not necessary to place reinforcement in the roof insulation layer. The roof insulation layer may comprise a reinforcement.

According to a further embodiment, the concrete roof structure comprises reinforcement. The spacers in the roof insulation layer are connected to the reinforcement in the concrete roof structure. As previously described, the concrete roof structure preferably comprises an upper reinforcement and a lower reinforcement. The upper reinforcement and the lower reinforcement are as previously described. The reinforcement meshes of the upper reinforcement and the lower reinforcement are advantageous for supporting the dead weight of the concrete of the concrete roof structure and an optional agitator. The spacers in the roof insulation layer are preferably connected to the lower reinforcement.

This embodiment provides better suspension of the roof insulation layer from the concrete roof structure. By connecting the spacers in the roof insulation layer to the reinforcement in the concrete roof structure, it is prevented that the part of the spacers protruding into the concrete roof structure is pulled out of the concrete roof structure by the weight of the roof insulation layer.

According to a preferred embodiment, the concrete roof structure rests on the concrete side wall. The roof insulation layer is placed entirely within the concrete tank. This embodiment is particularly advantageous in the case of a roof insulation layer formed in place. The wall of the tank thus serves as a formwork for the insulation formed on site. By using the already present concrete side wall as formwork when spraying roof insulation in place, the number of required formwork elements can be reduced, resulting in a simplification of the construction process and a reduction in material and labor costs. Moreover, by spraying directly against the concrete side wall, an improved connection is achieved between the insulation layer and the concrete structure, thereby limiting thermal leaks and cold bridges and increasing structural integrity. No cutting losses occur when fitting the boards to size. In addition, the concrete side wall provides stable and robust support during spraying, which increases the positioning accuracy of the insulation layer and reduces the risk of shifting or defects. Since spraying insulation material can cause significant internal pressures, the concrete side wall forms a necessary solid boundary that can withstand these pressures without deformation or damage to the structure. The insulation layer can be installed without interruption and immediate casting ensures good and structural integration. In addition, the roof insulation can be mechanically anchored to the roof structure via lattice girders, resulting in a strong and durable connection between the insulation layer and the roof structure. The plastic lining can be anchored in the insulation layer via the protrusions without gluing or fastening. The roof insulation layer is then preferably suspended from the concrete roof structure according to a previously described embodiment. This embodiment also has the advantage that the concrete tank has a simple structure.

According to an alternative embodiment, the concrete roof structure rests on an outer part of the concrete side wall and the roof insulation layer rests on an inner part of the concrete side wall. The outer part refers to a part of a top side of the concrete side wall that is closest to an exterior side of the concrete tank. Logically, the inner part is then a part of the top side of the concrete side wall that is closest to an interior side of the concrete tank. This embodiment is advantageous in the case of a roof insulation layer with boards (because these can then be more easily cut to fit the curvature). In combination with a previously described plastic lining, the plastic lining can also rest on the inner part of the concrete side wall. However, this is not necessary. The plastic lining under the roof insulation layer could also be limited to an interior side of the concrete tank.

According to a preferred embodiment, the roof insulation layer comprises a cured insulating concrete mortar. The insulating concrete mortar comprises expanded polystyrene granules. Such a cured concrete mortar is also known as EPS concrete, where EPS stands for Expanded Polystyrene. Insulating concrete mortar is advantageous because a high insulation value is achieved with a minimal layer thickness. The insulating concrete mortar is additionally advantageous because the roof insulation layer can be formed in place, for example in the formwork for casting the concrete roof structure. The insulating concrete mortar is also very suitable for installing spacers according to a previously described embodiment, with which the insulating concrete mortar is suspended from the concrete roof structure. The primary function of the insulating concrete mortar is to insulate the concrete tank and it does not necessarily contribute to the structural strength of the concrete tank. The spacers in the cured insulating concrete mortar can be part of a reinforcement to absorb tensile stresses in the cured insulating concrete mortar, but as previously described, this is not the primary function. This embodiment can advantageously be combined with an embodiment in which a plastic lining is installed under the roof insulation layer, preferably a plastic lining comprising protrusions.

According to an alternative embodiment, the roof insulation layer comprises a polyurethane insulating foam layer. Polyurethane insulating foam is very light and results in a high insulation rating. The use of polyurethane insulating foam results in similar advantages as a cured insulating concrete mortar. Polyurethane insulating foam has the disadvantage compared to insulating concrete mortar that favorable weather conditions are necessary to form the roof insulation layer in place. For example, it must not rain and it is preferably windless. This embodiment can advantageously be combined with an embodiment in which a plastic lining is installed under the roof insulation layer.

According to an embodiment, the roof insulation layer has a thickness of at least 10 mm, preferably at least 15 mm, and more preferably at least 20 mm. The roof insulation layer preferably has a thickness of at most 100 mm, more preferably at most 90 mm, and even more preferably at most 80 mm. A roof insulation layer of at least 10 mm provides sufficient insulation for the roof of the concrete tank, while a thickness of at most 300 mm avoids an unnecessarily thick insulation layer.

In a second aspect, the invention relates to a method for manufacturing a concrete tank for fermentation.

The method comprises the steps of:
- casting a concrete floor slab,
- casting a concrete side wall,
- casting a concrete roof structure.

The concrete floor slab is preferably cast in place. The concrete floor slab is preferably reinforced using reinforcement meshes. Preferably, the floor slab comprises an upper reinforcement and a lower reinforcement. The concrete floor slab is cast directly onto a ground surface or onto a foundation. The foundation is a trench or pile foundation.

The concrete side wall is preferably cylindrical. The concrete side wall is preferably cast in place. The concrete side wall is preferably cast layer by layer. The concrete side wall is preferably reinforced. The concrete side wall is reinforced using reinforcement meshes. Alternatively, the concrete side wall is reinforced with pre-tensioned or post-tensioned cables, wherein the pre-tensioned or post-tensioned cables are placed in circular loops in the concrete side wall. Alternatively, the concrete sidewall is reinforced with a combination of at least two elements from a group consisting of reinforcement meshes, pre-tensioned cables, and post-tensioned cables. The concrete side wall is preferably insulated.

The concrete roof structure is preferably reinforced.

The concrete roof structure is cast in place. For this, a temporary scaffolding is built inside the concrete tank to support a formwork for casting the concrete roof structure. This formwork is less complex and therefore easier to install, making this construction faster and cheaper to install.

According to a preferred embodiment, after casting the concrete side wall, a formwork for casting the concrete roof structure is installed. A roof insulation layer is installed in the formwork. The concrete roof structure is cast onto the roof insulation layer. The roof insulation layer is, for example, constructed with pre-formed boards made of extruded polystyrene, polyurethane, polyisocyanurate, or another suitable material. Preferably, the roof insulation layer is formed in place, as described in other embodiments. By casting the concrete roof structure onto the roof insulation layer, the roof insulation layer and the concrete roof structure are bonded together.

This method has, among other things, the advantage that by placing the roof insulation layer in the formwork, no additional insulation, for example in the form of sandwich panels, needs to be installed on the concrete roof structure afterwards. As a result, it is also not necessary to install walkways on the roof of the concrete tank to make the roof accessible, further simplifying the construction of the concrete tank. An additional advantage of the insulation on the interior side is that no extra damage can occur during movement. An additional advantage is that, depending on the chosen insulation material, the roof insulation layer already provides initial protection against sulfur or other aggressive substances in the methane gas formed in the concrete tank.

According to a preferred embodiment, before installing the roof insulation layer in the formwork, a plastic lining for protecting the concrete roof structure and the roof insulation layer is placed in the formwork. The plastic lining is, for example, in the form of a foil. The foil can be, without being limited thereto, one of the following types: a foil provided with large V-shaped protrusions that promote anchoring in the cast insulation layer; a foil provided with small loop-shaped protrusions that enable bonding with a (adhesive) mortar layer between the foil and the insulation boards or between the foil and a concrete intermediate layer; or a foil whose upper side is finished with a non-woven structure that also ensures bonding with the (adhesive) mortar or the concrete intermediate layer. A non-limiting list of suitable materials for the plastic lining includes polyethylene, polyvinyl chloride, polypropylene. The roof insulation layer is preferably applied directly to the plastic lining. An adhesive layer may optionally be applied to the plastic lining for gluing the plastic lining to the roof insulation layer. Alternatively, the roof insulation layer is formed in place. By forming the roof insulation layer in place, the roof insulation layer and the plastic lining are bonded together. Alternatively, an additional concrete layer is cast between the roof insulation layer and the plastic lining, with the additional concrete layer preferably cast directly onto the plastic lining. This is advantageous if the plastic lining and the roof insulation layer are difficult to bond together. By casting the additional concrete layer onto the plastic lining, the plastic lining and the additional concrete layer are bonded together. The plastic lining may comprise protrusions to promote bonding of the plastic lining to the roof insulation layer or the additional concrete layer. In a further embodiment, the concrete roof structure forms an almost flat roof with a slight slope for drainage. Thanks to this slope, rainwater does not remain on the roof, while the flat structure makes it possible to access and walk on the roof. Moreover, this roof surface can be used as additional space for installing equipment, so that the roof serves a dual function: both as a structural closure element and as a functional space for technical applications.

This embodiment is particularly advantageous because the plastic lining, regardless of the chosen insulation material, protects the concrete roof structure against sulfur or other aggressive substances in the methane gas or other gases formed in the concrete tank.

According to a preferred embodiment, before installing the roof insulation layer in the formwork, spacers are installed in the formwork. The spacers are preferably lattice girders. Non-limiting examples of suitable lattice girders are support girders, A-girders, rebar chairs, zigzag girders, etc. After installing the spacers in the formwork, the layer of roof insulation is sprayed or cast into the formwork around the spacers. The spacers protrude above the roof insulation layer. The spacers preferably protrude at least 20 mm above the roof insulation layer, more preferably at least 25 mm, even more preferably at least 30 mm, even more preferably at least 35 mm, and most preferably at least 40 mm. Then the concrete roof structure is cast around the spacers that protrude above the roof insulation layer.

This embodiment is particularly advantageous in combination with a roof insulation layer formed in place. The spacers in the roof insulation layer will protrude into the concrete roof structure, so that the roof insulation layer will automatically be firmly attached to the concrete roof structure.

According to a further embodiment, the method comprises the additional step of installing reinforcement for reinforcing the concrete roof structure. The reinforcement is connected to the spacers that protrude above the roof insulation layer before casting the concrete roof structure. The spacers in the roof insulation layer are preferably connected to a lower reinforcement for the concrete roof structure. Preferably, the spacers are connected to the reinforcement of the concrete roof structure before installing the roof insulation layer. As a result, the installation of the reinforcement and the spacers can be carried out as a single step.

This embodiment provides better suspension of the roof insulation layer from the concrete roof structure. By connecting the spacers in the roof insulation layer to the reinforcement in the concrete roof structure, it is prevented that the part of the spacers protruding into the concrete roof structure is pulled out of the concrete roof structure by the weight of the roof insulation layer.

According to a preferred embodiment, a layer of insulating concrete mortar is cast in the formwork. After curing, the layer of insulating concrete mortar forms the roof insulation layer. The insulating concrete mortar comprises expanded polystyrene granules. Insulating concrete mortar is advantageous because a high insulation value is achieved with a minimal layer thickness. The insulating concrete mortar is also very suitable for installing spacers according to a previously described embodiment. This embodiment can advantageously be combined with an embodiment in which a plastic lining is installed under the roof insulation layer, preferably a plastic lining comprising protrusions, as described in the first aspect.

According to an alternative embodiment, a layer of polyurethane insulating foam is sprayed in the formwork. The layer of polyurethane insulating foam forms the roof insulation layer. Polyurethane insulating foam is very light and results in a high insulation rating. The use of polyurethane insulating foam results in similar advantages as insulating concrete mortar. Polyurethane insulating foam has the disadvantage compared to insulating concrete mortar that favorable weather conditions are necessary to form the roof insulation layer in place. This embodiment can advantageously be combined with an embodiment in which a plastic lining is installed under the roof insulation layer.

A person skilled in the art will appreciate that a method according to the second aspect is preferably carried out for manufacturing a concrete tank according to the first aspect and that a concrete tank according to the first aspect is preferably manufactured by carrying out a method according to the second aspect. Each feature described in this document, both above and below, can therefore relate to any of the three aspects of the present invention.

In a third aspect, the invention relates to a use of a concrete tank according to a first aspect for pre-acidification and/or biomass fermentation.

This use results in a cost-effective biomass fermentation in a concrete tank, whereby a high and stable temperature is obtained in the concrete tank due to the layer of roof insulation. As a result, little or no heat needs to be supplied to the concrete tank to maintain the fermentation or pre-acidification. A stable climate can be achieved in the concrete tank.

In what follows, the invention is described by way of non-limiting figures illustrating the invention, and which are not intended to and should not be interpreted as limiting the scope of the invention.

### DESCRIPTION OF THE FIGURES

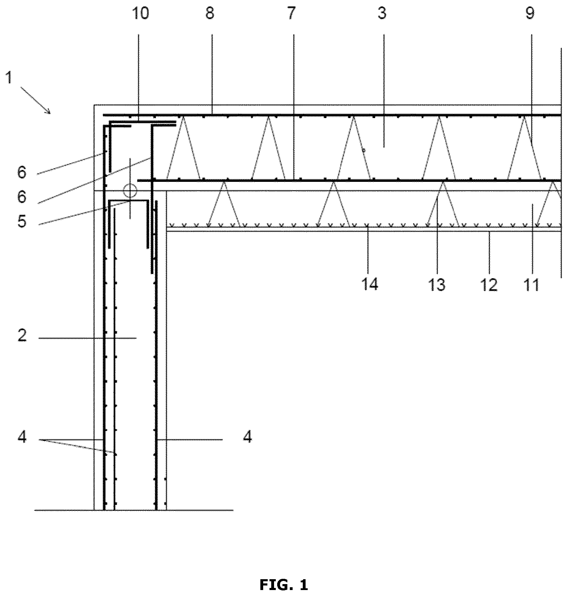

**Figure 1** shows a cross-section in a vertical plane of a concrete roof structure of a concrete tank according to an embodiment of the present invention.

The concrete tank (1) comprises a concrete floor slab (2), a cylindrical concrete side wall (2), and a concrete roof structure (3). Figure 1 only shows a detail of the corner between the cylindrical concrete side wall (2) and the concrete roof structure (3). The concrete floor slab is not shown. The cylindrical concrete side wall (2) comprises reinforcement meshes (4) and, at a top side, U-shaped reinforcement bars (5) for reinforcing the concrete side wall (2). The cylindrical concrete side wall (2) also comprises, at the top side, L-shaped reinforcement bars (6) for connecting the cylindrical concrete side wall (2) to the concrete roof structure (3). The concrete roof structure (3) comprises a first reinforcement mesh as lower reinforcement (7) and a second reinforcement mesh as upper reinforcement (8). The lower reinforcement (7) and the upper reinforcement (8) are kept at a fixed distance from each other by spacers (9) before and during casting of the concrete roof structure (3). The spacers (9) in this embodiment are lattice girders, more specifically A-girders. The concrete roof structure (3) comprises, at an edge, L-shaped reinforcement bars (10) for reinforcing the edge. The concrete roof structure (3) has a thickness of 350 mm in this embodiment. The lower reinforcement (7) is placed 40 mm from an underside and the upper reinforcement (8) 40 mm from a top side of the concrete roof structure (2). Under the concrete roof structure (3) is a roof insulation layer (11) and below that a plastic lining (12). The roof insulation layer (11) and the plastic lining (12) are placed entirely within the concrete tank (1) and the concrete roof structure (3) rests on the concrete side wall (2). The roof insulation layer (11) comprises spacers (13). The spacers (13) protrude above the roof insulation layer (11) into the concrete roof structure (3) and are connected to the lower reinforcement (7). The spacers (13) in this embodiment are lattice girders, more specifically A-girders. The roof insulation layer (11) in this embodiment comprises a polyurethane insulating foam or a cured insulating concrete mortar comprising expanded polystyrene granules. The roof insulation layer (11) in this embodiment has a thickness of 150 mm. The spacers (13) protrude 40 mm above the roof insulation layer (11). The plastic lining (12) in this embodiment is a foil made of polyvinyl, polyethylene, or polypropylene. The plastic lining (12) comprises V-shaped protrusions (14) that engage in the roof insulation layer (11). The plastic lining (12) in this embodiment has a thickness of 15 mm. The plastic lining (12) is placed first in a formwork for casting the concrete roof structure (3). Then the spacers (13) are placed on the plastic lining (12), after which the roof insulation layer (11), in the case of polyurethane insulating foam, is sprayed onto the plastic lining (12) or, in the case of insulating concrete mortar, is cast onto the plastic lining (12). Then the concrete roof structure (3) is cast onto the roof insulation layer (11).

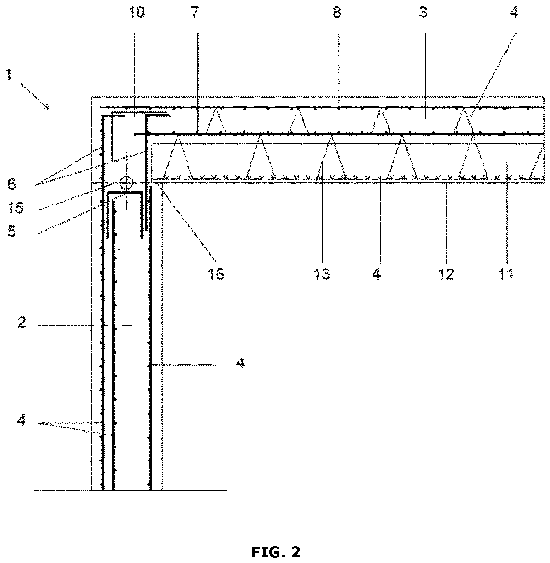

**Figure 2** shows a cross-section in a vertical plane of a concrete roof structure of a concrete tank according to an alternative embodiment of the present invention.

This alternative embodiment is very similar to the embodiment in Figure 1. The main difference is that the concrete roof structure (3) rests on an outer part (15) of the concrete side wall (2) and the roof insulation layer (11) and the plastic lining (12) rest on an inner part (16) of the concrete side wall (2). The roof insulation layer (11) and the plastic lining (12) have the same thickness as the embodiment in Figure 1. The concrete roof structure (3) has a thinner thickness of 200 mm above the roof insulation layer (11) and the plastic lining (12).

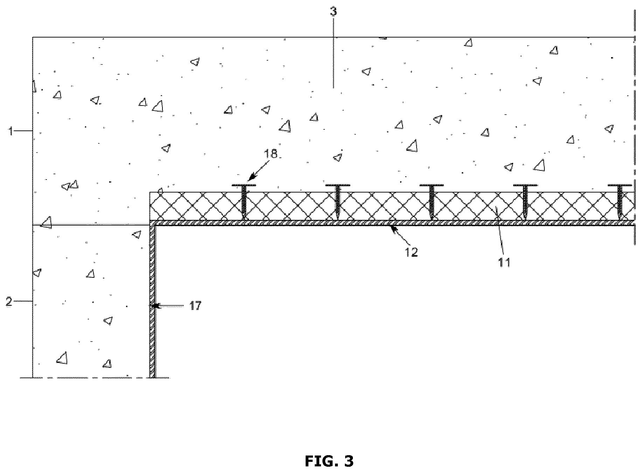

Figure 3 shows a cross-section in a vertical plane of a concrete roof structure of a concrete tank according to an alternative embodiment of the present invention.

This alternative embodiment is very similar to the embodiment in Figure 2. In this embodiment, the concrete roof structure (3) rests on an outer part (15) of the concrete side wall (2). The difference with Fig. 2 is that the roof insulation layer (11) consists of pre-formed insulation boards on which the concrete roof structure (3) can then be cast. In this embodiment, the roof insulation layer (11) comprises a waffled structure suitable for improved interaction between the roof insulation layer (11) and the concrete roof structure (3). In this alternative embodiment, the roof insulation layer (11) also includes optional PVC nails (18). These nails are installed when applying the roof insulation layer (11) and before casting the roof structure (3). These nails provide additional anchoring of the roof insulation layer (11) in the roof structure (3). This anchoring is achieved by the larger head of the nails. The roof insulation layer (11) is an XPS board onto which concrete can be cast immediately without the step required for sprayed insulation to dry first. The advantage here is that XPS has high compressive strength and moisture resistance. The use of pre-made insulation boards can be advantageous because it does not require insulation material to be sprayed or cast in place, making the process less dependent on environmental conditions such as temperature, humidity, and substrate condition. For spray or casting applications, specific conditions are required to obtain a reliable and homogeneous insulation assembly, which in practice is not always easy to achieve. By using pre-formed boards, the risk of defects such as air pockets, bonding problems, or material faults can be reduced. Moreover, the use of boards can contribute to faster execution and increased reproducibility of the insulation layer quality. In addition, it is not necessary to use spacers and the like, which are required when spraying the insulation layer in place. The roof insulation layer (11) and the plastic lining (12) have the same thickness as the embodiment in Figure 1. To achieve better bonding between the plastic lining (12) and the roof insulation layer (11), a layer of bonding adhesive or mortar (17) is used, which ensures good adhesion between the two layers. The adhesive can be applied before casting the concrete, for example at the time the plastic lining is attached to the formwork, or afterwards, after the concrete side wall has been cast. In both cases, the adhesive contributes to improved bonding of the plastic lining to the concrete surface. Other types of chemical compounds can also be used to improve adhesion. In addition to the roof insulation layer (11), the side wall also comprises the plastic lining (12) that uses the bonding mortar (17), although this mortar is not essential to the invention. The non-essential bonding mortar can optionally provide improved bonding between the protrusions of the plastic lining (12) and the concrete side wall (2). By using a bonding mortar (17), side walls can be easily lined with a plastic lining (17) after installation, resulting in a seamless finish of the interior wall of the concrete tank. This not only increases protection against chemical degradation and moisture penetration, but also reduces the risk of leaks and facilitates cleaning and maintenance of the tank.

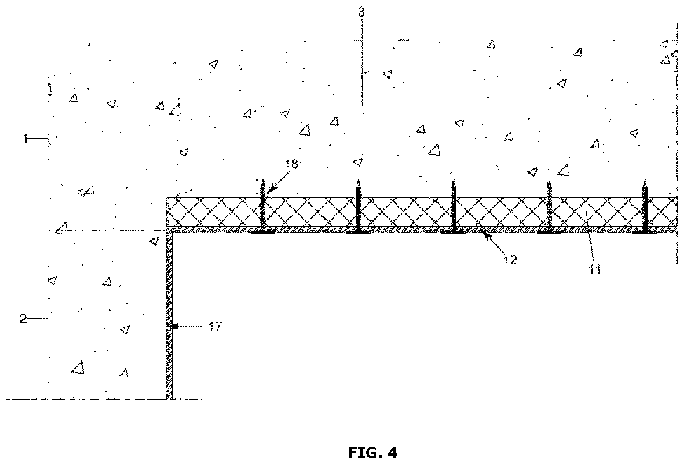

Figure 4 shows a cross-section in a vertical plane of a concrete roof structure of a concrete tank according to an alternative embodiment of the present invention.

The main difference of this embodiment from the embodiment of figure 3 is that insulation plugs are used that run through both the plastic lining (12) and the roof insulation layer (11). As a result, both layers are mechanically anchored to the concrete roof structure (3). The insulation plugs are designed so that the heads have a significantly larger surface area, so that the plastic lining (12) will tear less quickly during use of the fermentation tank. This embodiment, like the embodiment in figure 3, has the advantage that the plastic lining (12) can be easily installed after placement, if necessary.

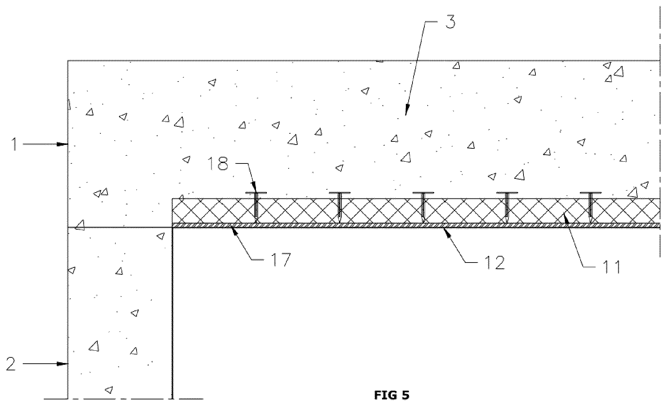

**Figure 5** shows a cross-section in a vertical plane of a concrete roof structure of a concrete tank according to an alternative embodiment with nails in the insulation and with plastic lining on the side wall.

This embodiment uses a plastic lining (12) that can immediately bond with the side wall via its protrusions and thus remains anchored. The plastic lining (12) is tensioned in the formwork before casting the concrete. This ensures seamless integration of the plastic lining. In this embodiment, a cast-in plastic lining (12) is used. The plastic lining (12) is applied and tensioned in the formwork before casting the concrete. As a result, the lining fits seamlessly against the interior of the final concrete structure. In this embodiment, a plastic lining (12) is used that is integrally cast into the concrete, resulting in a durable, structurally anchored interior lining (11). Here, the anchoring nails (18) are inserted into the insulation boards of the roof insulation layer (11) onto which the roof structure is then cast. The anchoring nails provide additional anchoring between the concrete roof structure (3) and the roof insulation layer (11).

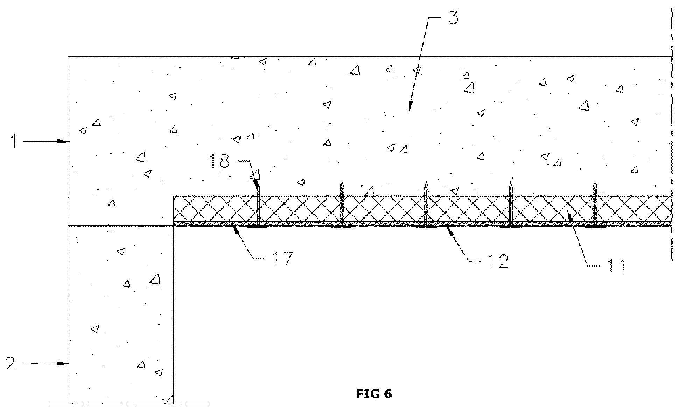

**Figure 6** shows a cross-section in a vertical plane of a concrete roof structure of a concrete tank according to an alternative embodiment with nails in the insulation and with plastic lining on the side wall.

This embodiment is similar to the embodiment of Figure 5, but the main difference is that the anchoring nails (18) are inserted into the roof insulation layer from the interior side. The plastic lining (12) is also applied beforehand here. This not only provides additional anchoring of the roof insulation layer (11) but also improved bonding of the plastic lining (12) to the roof insulation layer (11).

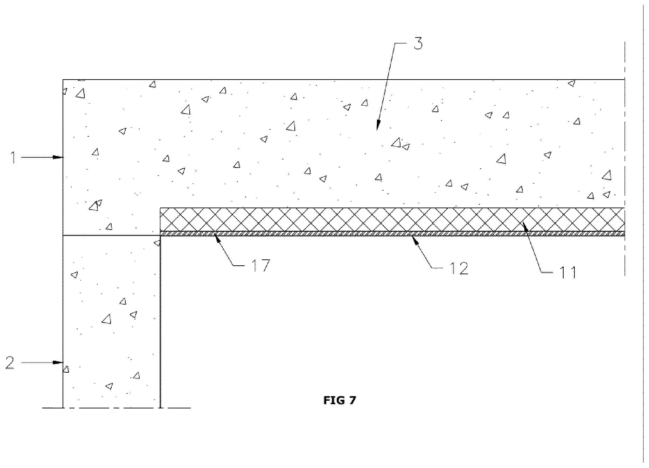

**Figure 7** shows a cross-section in a vertical plane of a concrete roof structure of a concrete tank according to an alternative embodiment with plastic lining on the side wall.

In this embodiment, no anchoring nails are used. This can be advantageous in applications where the protrusions of the plastic lining (12) are sufficiently strongly bonded to the roof insulation layer (11) and the bonding mortar (17).

The numbered elements in the figures are:
1. Concrete tank
2. Concrete side wall
3. Concrete roof structure
4. Reinforcement mesh
5. U-shaped reinforcement bar of concrete side wall
6. L-shaped reinforcement bar of concrete side wall
7. Lower reinforcement
8. Upper reinforcement
9. Spacer concrete roof structure
10. L-shaped reinforcement bar of concrete roof structure
11. Roof insulation layer
12. Plastic lining
13. Spacer roof insulation layer
14. V-shaped protrusion
15. Outer part of concrete side wall
16. Inner part of concrete side wall
17. Bonding mortar
18. Anchoring nail

## Claims

1. Concrete tank for fermentation comprising a concrete floor slab, a concrete side wall, and a concrete roof structure, the concrete tank comprising a roof insulation layer, wherein the roof insulation layer is placed on an interior side of the concrete tank against the concrete roof structure, **characterized in that** a plastic lining for protecting the concrete roof structure and the roof insulation layer is installed on the interior side of the concrete tank under the roof insulation layer.

2. The concrete tank according to claim 2, **characterized in that** the plastic lining comprises protrusions, wherein the protrusions engage in the roof insulation layer.

3. The concrete tank according to any of the preceding claims 1-3, **characterized in that** the roof insulation layer comprises spacers, wherein the spacers protrude above the roof insulation layer into the concrete roof structure.

4. The concrete tank according to claim 4, **characterized in that** the concrete roof structure comprises reinforcement, wherein the spacers in the roof insulation layer are connected to the reinforcement in the concrete roof structure.

5. The concrete tank according to any of the preceding claims 1-5, **characterized in that** the concrete roof structure rests on the concrete side wall, wherein the roof insulation layer is placed entirely within the concrete tank.

6. The concrete tank according to any of the preceding claims 1-5, **characterized in that** the concrete roof structure rests on an outer part of the concrete side wall and the roof insulation layer rests on an inner part of the concrete side wall.

7. The concrete tank according to any of the preceding claims 1-7, **characterized in that** the roof insulation layer comprises a polyurethane insulating foam layer.

8. The concrete tank according to any of the preceding claims 1-7, **characterized in that** the roof insulation layer comprises a cured insulating concrete mortar, wherein the insulating concrete mortar comprises expanded polystyrene granules.

9. Method for manufacturing a concrete tank for fermentation comprising the steps of:
- casting a concrete floor slab;
- casting a concrete side wall;
- casting a concrete roof structure;
**characterized in that** after casting the concrete side wall, a formwork for casting the concrete roof structure is installed, wherein a roof insulation layer is installed in the formwork, after which the concrete roof structure is cast onto the roof insulation layer.

10. The method according to claim 10, **characterized in that** before installing the roof insulation layer in the formwork, a plastic lining for protecting the concrete roof structure and the roof insulation layer is placed in the formwork.

11. The method according to claim 10 or 11, **characterized in that** before installing the roof insulation layer in the formwork, spacers are installed in the formwork, after which a roof insulation layer is sprayed or cast in the formwork around the spacers, wherein the spacers protrude above the roof insulation layer and after which the concrete roof structure is cast around the spacers that protrude above the roof insulation layer.

12. The method according to claim 12, **characterized in that** the method comprises the additional step of installing reinforcement for reinforcing the concrete roof structure, wherein the reinforcement is connected to the spacers that protrude above the roof insulation layer before casting the concrete roof structure.

13. The method according to any of claims 10-13, **characterized in that** a layer of polyurethane insulating foam is sprayed in the formwork.

14. The method according to any of claims 10-13, **characterized in that** a layer of insulating concrete mortar is cast in the formwork, wherein the insulating concrete mortar comprises expanded polystyrene granules.

15. Use of a concrete tank according to any of claims 1-9 for pre-acidification and/or biomass fermentation.
